# EUROPÄISCHE PATENTSCHRIFT

(11) **EP 0 940 117 B1**
(45) Veröffentlichungstag und Bekanntmachung des Hinweises auf die Patenterteilung: **15.08.2007**
(21) Anmeldenummer: 99104439.7
(22) Anmeldetag: 05.03.1999
(51) Int. Cl.: A61B 3/08, A61B 3/18

(54) **Verfahren und Anlage zum Durchführen von Seh-Untersuchungen**
Method and apparatus for carrying out vision tests
Méthode et appareil de mise en oeuvre d'examens de la vision

(30) Priorität: 06.03.1998 DE 19809591
(43) Veröffentlichungstag der Anmeldung: 08.09.1999
(73) Patentinhaber: Neuhof, Judith, 35452 Giessen-Heuchelheim (DE); Neuhof, Steffen, 35452 Giessen-Heuchelheim (DE)
(72) Erfinder: Neuhof, Judith, 35452 Giessen-Heuchelheim (DE); Neuhof, Steffen, 35452 Giessen-Heuchelheim (DE)
(74) Vertreter: Olbricht, Karl Heinrich

(56) Entgegenhaltungen:
- WO-A-90/01290
- WO-A-91/00050
- WO-A-96/13195
- DE-A- 3 808 420
- DE-A- 4 417 768
- DE-A- 19 500 272
- DE-A- 19 505 399
- DE-A- 19 543 973
- FR-A- 2 425 684
- US-A- 5 311 879

## Beschreibung

Die vorliegende Erfindung betrifft ein Verfahren und eine Anlage zum Durchführen von Seh-Untersuchungen gemäß den Oberbegriffen der Ansprüche 1 und 21.

Für Untersuchungen z.B. von Sehstörungen wie Strabismus benutzt man seit langem Systeme der Koordimetrie, wobei der Kopf fixiert wird und nur die Augen bewegt werden. Man mißt horizontale und vertikale Deviationen nach dem Konfusionsprinzip in neun standardisierten Blickrichtungen.

An einer Tangententafel wie der Harms-Wand können Blickrichtungen umgekehrt bei fest auf ein Fixierlicht gerichtetem Auge mit wechselnder Kopfhaltung beobachtet werden. Das ermöglicht die Untersuchung auch von Zyklo-Deviationen sowie der Exkursionsfähigkeit und des Fusionsblickfeldes. Ausschließlich bei 45° kann ferner der Bielschowsky-Kopfneigtest durchgeführt werden.

Beide Untersuchungsarten sind aber grundsätzlich nur anwendbar bei Patienten mit normaler Netzhautkorrespondenz, die gleichzeitig Doppelbilder haben; dies ist eine Minderheit aller Schielpatienten.

Der Untersuchungsschirm ist typisch eine großflächige Tafel aus festem Material, die Koordinatenskalen gemäß augenärztlichen Berechnungen trägt; daher stammen Bezeichnungen wie Harms-Wand, Helmholtz-Schirm, Hess-Schirm, Lee-Screen, Maddox-Kreuz usw. Ein Koordimetrieschirm weist verschieden stark gekrümmte, sich schneidende Kurvenscharen auf. Während der Messung, die allgemein einen festen Betrachterabstand zwischen Untersuchungsschirm und Proband erfordert, sind dessen Blickrichtungen jeweils einer Seheindrucks-Position zugeordnet. Deshalb wird der Begriff Tangententafel im Sinne der vorliegenden Anmeldung auf alle Untersuchungsschirme einschließlich solcher der Koordimetrie angewandt.

Bei der Untersuchung muß ein Proband mit Leuchtzeigern angeben, wo er auf dem Schirm vor sich einen bestimmten Seheindruck wahrnimmt; das ist wegen des naturgegebenen Handzitterns immer etwas ungenau. Auch Ablesungen und Meßwert-lnterpolationen sind individuell verschieden. Für den Untersucher ist es oft anstrengend, die Kopfstellung zu kontrollieren, vor das fixierende Auge ein Farbfilter zu halten, angezeigte Positionen abzulesen, sie gegebenenfalls umzurechnen und schließlich für jede untersuchte Blickrichtung zu protokollieren. Nachteilig waren bisher ferner Diagnose-Abweichungen aufgrund unterschiedlicher Untersuchungsverfahren und -bedingungen.

Ein aus DE-C-40 41 332 bekannter Schirm hat Leuchtdioden im Zentrum und an Schnittpunkten sich kreuzender Linien, die für einen einzigen Betrachterabstand berechnet sind, Leuchtdioden zur Markierung von Festpunkten. Diese können etwa mittels einer Fernbedienung nach DE-U-92 601 910 angesteuert und aktiviert werden. Um die Aufzeichnung zusätzlicher Untersuchungswerte zu erleichtern, schlägt DE-U-296 19 371 eine Art Steuerpult vor, das an bzw. in einem flachen Gehäuse ein Tastenfeld und ein oder mehrere Leuchtfelder sowie eine Auflage für ein Aufzeichnungsschema aufweist, in das der Untersucher abgelesene Positionen von Hand einträgt.

Eine in DE-C-38 08 420 beschriebene Untersuchungs-Anordnung benutzt einen Projektor, der in festem Abstand ein grünes Dia-Raster als Tangententafel auf eine Fläche (Hess-Schirm oder Harms-Wand) projiziert. Ein Proband, der verschiedene Vorhalter trägt, z.B. eine Rot-Grün-Brille, versucht subjektiv gesehene Positionen eines vom Untersucher vorgegebenen weißen Fixierpunkts (oder Striches) mit einem Rotlicht-Handprojektor zu markieren. Das ist ebenso wie die Protokollier-Tätigkeit mit Unsicherheiten verbunden. Eine automatische Erfassung und Auswertung von Meßergebnissen ist mit dieser Anordnung nicht möglich. Das Projektions-Raster ist für nur einen Untersuchungs-Abstand berechnet.

Gemäß WO 96/13195 benutzt man zum Bestimmen von Deviationen am Auge weißes Fixierlicht im Zentrum eines Maddox-Kreuzes oder einer Harms-Wand, wohin Positionslicht von einem Stirnprojektor am stillgehaltenen Kopf eines Probanden gerichtet werden kann. Dabei wird jeweils ein Auge z.B. mit einem Rotglas abgedeckt, um eine Seheindrucks-Fusion zu unterbinden, während der Proband einen z.B. grünen Zeigestrahl auf die Stelle richtet, wo er das rote Fixierlicht sieht. Wenn man für die Änderung der Blickrichtung eine Strahlenteiler-Anordnung einsetzt und über eine Umlenkoptik punkt- oder linienförmiges Fixierlicht auf einer Tafel im Blickfeld des Probanden abbildet, wird für die Fusions-Unterdrückung eine Polarisationsfilterung verwendet. Die jeweiligen Fixierobjekte werden jedoch stets durch eine Optik hindurch an Stellen gesehen, an denen sie sich in Wirklichkeit nicht befinden. Die mittels eines Steuerblocks, einer Hand-Fernbedienung, eines Tastenfeldes o.dgl. gesteuerten Meßwerte werden auf einem Display angezeigt und sind gegebenenfalls über ein Computerprogramm auswertbar.

Herkömmlich wurden bei Systemen, die Visustests oder andere Sehtests ermöglichen, die Meßwerte nicht zu Blickrichtungen über einen Betrachterabstand in Beziehung gesetzt. Die Menge untersuchbarer Blickrichtungen war durch eine endliche Anzahl vorgegebener Blickpunkte beschränkt. Vielfach konnten, z.B. um einen Monitor herum, irritierende Seheindrücke nicht oder nicht befriedigend ausgeschaltet werden. Örtliche Raumgegebenheiten begrenzten den praktisch möglichen Betrachterabstand und die davon abhängige Größe von Tangententafeln, da deren Abmessungen ja mit zunehmendem Betrachterabstand wachsen. Bei kleinem Format resultiert zwangsläufig ein geringer Betrachterabstand, was zu unakzeptablen Akkommodations- und Konvergenzfehlern führte; daran scheiterten bislang Versuche einer Nachbildung auf Bildschirmen. Insbesondere bei gewölbten Bildröhren maximierten sich Abstandsfehler, und schon kleinste Änderungen der Kopfhaltung bewirkten unverhältnismäßige Fehlerquellen. Auch traten bei großen Schielwinkeln (über 30°) beispielsweise an der Harmswand erhebliche Fehler auf, weil der Untersucher die Winkel mit Näherungen ablesen, d.h. interpolieren mußte.

Aufgabe der Erfindung ist es, diese und weitere Nachteile des Standes der Technik mit wirtschaftlichen Mitteln zu überwinden und verbesserte Möglichkeiten für Seh- Untersuchungen zu schaffen, die schneller und weniger anstrengend vor sich gehen können. Bei gleichen Betrachterabständen wird ein erweitertes Blickfeld angestrebt. Ferner sollen mit ein und denselben Mitteln zusätzliche Untersuchungen ausgeführt werden können, die bislang gesonderte Apparaturen oder Umbauten erforderten. Ein wichtiges Ziel der Erfindung ist es, die zu erzielenden Meßergebnisse reproduzierbar und sowohl genauer als auch zuverlässiger als bislang zu machen. Außerdem soll die Protokollierung automatisierbar sein.

Hauptmerkmale der Erfindung sind in den Ansprüchen 1 und 21 angegeben. Ausgestaltungen sind Gegenstand der Ansprüche 2 bis 20 und 22 bis 36.

Zum Durchführen von Seh- Untersuchungen, insbesondere bei Probanden-Sehstörungen wie Strabismus, unter Verwendung von Projektions-Einrichtungen, Seheindruck-Gebern, Fixier- und Zeigeobjekten, Tangententafeln sowie elektronischen und computerunterstützten Meß- und Auswertemitteln, mit Überwachung der Kopf- und/oder Augapfel-Position, sieht die Erfindung ein Verfahren gemäß Anspruch 1 und eine Anlage gemäß Anspruch 20 vor, die sich dadurch auszeichnen, daß auf einer Darstellungsfläche rechnergesteuert eine virtuelle Tangententafel erzeugbar ist, zu der koordinatenbezogene Seheindrücke freisichtig dargeboten werden.

Mithin ist eine körperliche Tangententafel mit fest vorgegebenen Abmessungen und Abständen nicht erforderlich; auch sind aufgezeichnete Linien unnötig, die den Betrachter irritieren konnten. Nunmehr lassen sich an ein und demselben Platz beliebige Tangententafeln, also visualisierte Koordinatensysteme, virtuell erzeugen. Im Sinne der Erfindung bedeutet 'virtuelle Tangententafel', daß ihre mathematisch-medizinischen Grundlagen rechnergebunden vorliegen und nach Bedarf in digitaler oder analoger Form abgerufen und visuell dargeboten, insbesondere projiziert werden können. Der Proband, welcher die Darstellungsfläche der virtuellen Tangententafel betrachtet, bekommt die Seheindrücke entsprechend digital oder analog. Er kann nur das sehen, was er für die Untersuchung tatsächlich sehen soll. Wenn allerdings ein Untersucher die Darbietung von Koordinatenlinien wünscht, lassen sie sich ohne weiteres zusätzlich projizieren. Zur Darbietung eignen sich nahezu beliebige Räumlichkeiten, so daß bislang gegebene Einschränkungen vor allem hinsichtlich Tangententafel-Größe und Betrachterabstand prinzipiell wegfallen. Durch die Abkehr von der Notwendigkeit gegenständlicher Tangententafeln wird eine außerordentliche Anlagenflexibilität erzielt und zugleich die Untersuchungsmethodik sowohl vereinfacht als auch qualitativ verbessert. Es lassen sich nun alle sensomotorischen Augenstörungen selbsttätig und in einem Gang feststellen. Ein weiterer Vorteil ist hohe Flexibilität der Untersuchung, da beliebige Meßpunkte und Tests zusätzlich eingefügt und abgespeichert werden können.

Laut Anspruch 2 ist vorgesehen, daß die Darstellungsfläche mit der virtuellen Tangententafel in einem den Probanden umgebenden Hüllraum angeordnet wird. Somit kann eine vorzugsweise flache Projektionsfläche vor, über, hinter, neben oder auch unter dem Betrachter erscheinen; sie kann ihn z.B. auf vorhandenen Raumflächen, Projektionswänden u.dgl. ganz oder teilweise umgeben, wobei sich allfällige Probleme eines Schattenwurfs durch geeignete Projektion mühelos beheben lassen. Welche Prüfdistanz gewählt wird, legt zweckmäßig der Untersucher fallweise fest.

Während die Untersuchung unter Rechnersteuerung automatisch ablaufen kann, ist es nach Anspruch 3 und Anspruch 22 auch möglich, Seheindrücke und Verfahrensschritte durch den Untersucher und/oder den Probanden interaktiv zu beeinflussen, namentlich mit elektronischen Hilfsmitteln einschließlich Fernbedienung, über Touchscreen, Tastenfeld, Steuerknüppel (Joystick), Mikrofon u.dgl., so daß mit geringem Aufwand eine erhebliche Beschleunigung des Untersuchungsablaufs erreichbar ist. Durch die genannten und weitere Komponenten wird auch für den Untersucher an der Anlage und bei der Durchführung der Untersuchungen ein Maximum an Bedienungsfreundlichkeit und guter Bedienerführung erreicht. Ferner erzielt man höhere Zuverlässigkeit, weil Unsicherheiten durch Handzittern entfallen und weil die Positionierung rechnergestützt erfolgt, so daß die Signale eines vom Probanden betätigten Stellgliedes genau umgesetzt und ausgewertet werden.

Im Einklang mit Anspruch 4 und Anspruch 23 ist die virtuelle Tangententafel und/oder sind die Seheindrücke auf einem Computermonitor, Großbildschirm o.dgl. erzeugbar. Es trägt erheblich zu großer Präzision bei und überschreitet die herkömmlichen Möglichkeiten bei weitem, wenn speziell nach Anspruch 5 Bilddaten eines Computers und/oder Videorecorders projiziert werden. Ferner eignen sich Video-, Daten- und Overheadprojektoren sowie durchleuchtbare Displays oder Chips. Auch eine Kombination solcher und noch anderer Projektoren ist möglich.

Vorteilhaft erzeugt man laut Anspruch 6 und Anspruch 24 die virtuelle Tangententafel und/oder die Seheindrücke mit Hilfe eines Projektionssystems durch Aufprojektion und/oder Rückprojektion, wobei exakt montierte Umlenkspiegel es erlauben, den Projektionsabstand auf gegebene Raumdimensionen einzustellen. Die Projektions-Einrichtungen können in einem Gehäuse vereinigt sein, bevorzugt in einer Rückprojektionsbox. Während die ProjektionsEinrichtung mit einer Umlenkoptik versehen sein kann, erfolgt die Untersuchung stets mit freisichtiger Betrachtung, d.h. ohne Zwischenschaltung von optischen Geräten. Wenn gemäß Anspruch 7 und Anspruch 25 zum Darbieten und/oder Positionieren von Seheindrücken auf einer Tangententafel ein Laserprojektor innerhalb des Hüllraumes (jedoch nicht an einer Kopfstütze oder als deren Bestandteil) vorhanden ist, werden die Untersuchungen durch die Intensität und Schärfe des Laserlichts noch genauer.

Sehr günstig ist es, daß man gemäß Anspruch 8 das Tangententafel-Koordinatensystem gemäß veränderlichen Parametern wie Ursprungsversetzung, Betrachter-Abstand, Probanden-Kopfhaltung usw. berechnen bzw. umrechnen kann, insbesondere in einer Rechnereinheit unter Verwendung von Algorithmen, Funktionsgleichungen u.dgl. Die benötigten Koordinaten und Kurven lassen sich jederzeit und außerordentlich schnell auf beliebige Betrachterabstände umrechnen. Die Berechnung ist im mathematischen Sinne infinitesimal bzw. quasi-kontinuierlich; infolgedessen entfallen Interpolationen, da für unbegrenzt viele Blickrichtungen immer genaue Blickrichtungspunkte zur Verfügung stehen. Auch für große Schielwinkel (z.B. über 30°) ist bei gleichbleibendem Betrachterabstand stets genügend Blickfeld durch eine entsprechende Ursprungsversetzung erzielbar; eine solche erlaubt außerdem eine physiologische Anpassung des Koordinatensystems. Da letzteres beliebig drehbar ist, kann man zudem den Bielschowsky-Kopfneigtest bei beliebigen Neigwinkeln ausführen. Dies ist ein besonderer Vorteil, weil nicht allen Patienten eine Kopfneigung um 45° möglich ist. Die Untersuchungen sind unter sonst gleichen Meßbedingungen in verschiedenen Entfernungen durchführbar.

Im Einklang mit Anspruch 9 und Anspruch 26 kann man das Koordinatensystem der virtuellen Tangententafel mit einem Hintergrundbild und/oder mit einer gegenständlichen Tangententafel kombinieren, wenn das etwa aufgrund vorhandener Einrichtungen erwünscht ist, so daß sich herkömmliche Untersuchungsmethoden besonders bequem mit der neuen Technik verbinden lassen. Mit einem Hintergrundbild beliebiger Art kann man den alltäglichen Sehbedingungen viel näherkommen als bisher und die gefragten Positionen noch genauer lokalisieren.

Wichtig ist, daß sich gemäß Anspruch 10 und Anspruch 27 in Verbindung mit der virtuellen Tangententafel beliebige Seheindrücke verwenden lassen. Dem System sind alle Seheindrücke nach Art und Position bekannt, und weil die gesamte Tangententafel virtuell existiert, können auch der Patientenzeiger und das Fixierobjekt virtuell dargestellt werden. Beliebige Seheindrücke waren für sich zwar bereits herkömmlich darstellbar; dies gilt aber nicht für Untersuchungen an Tangententafeln. Hier ermöglicht die Erfindung durch virtuelles Generieren erstmals die Darbietung auch anderer Zeichen als Punkte, Striche, Balken und Ringe. Mithin lassen sich koordimetrisch auch Zyklo-Deviationen bei fixiertem oder geradegehaltenem Kopf in beliebigen Blickrichtungen messen. Besonders günstig ist, daß Kinder auf spielende Weise untersucht werden können, was die Kooperations-Bereitschaft fördert oder überhaupt erst schafft.

Von besonderer Bedeutung ist die in Anspruch 11 und Anspruch 28 angegebene Möglichkeit einer automatischen Protokollierung mit Mitteln zur grafischen und/oder numerischen Aufzeichnung und Wiedergabe. Durch bloße Wahl eines vom Arzt oder einer Klinik praktizierten Ausdrucksystems kann man mühelos eine optimale Anpassung an den Usus erreichen. Dies macht die Registrierung und Ausgabe der Untersuchungs-Ergebnisse nicht nur für alle Beteiligten wesentlich angenehmer, sondern auch rascher. Dank der automatischen Protokollierung, die nach dem Stand der Technik nicht möglich war, kann sich der Untersucher völlig auf den Probanden bzw. dessen Angaben konzentrieren; zudem werden Ablesefehler vermieden. Kontrolluntersuchungen werden leichter, mit automatisch gegebenem Vergleich. Überaus vorteilhaft ist, daß die Untersuchungsergebnisse mit geeigneter Software direkt in eine Behandlungs-Empfehlung oder eine Operations-Indikation umgesetzt werden können.

Beachtliche Vorteile erzielt man laut Anspruch 12 dadurch, daß mit der Untersuchung an einer virtuellen Tangententafel andere Sehprüfungen z.B. des monokularen oder binokularen Sehens, Visustest, Stereotest usw. kombiniert werden können, und zwar auch bei nicht fixiertem Kopf in verschiedenen Blickrichtungen. Die Sehzeichen können über das gesamte Blick- und Sichtfeld hinweg bewegt werden, was den alltäglichen Blicksituationen am ehesten entspricht. Damit lassen sich die Sehprüfungen auch blickrichtungsabhängig durchführen. Bislang waren z.B. Kopfzwangshaltungen höchstens unzulänglich erfaßbar, und an Tangententafeln konnten nur Licht- oder Leuchtpunkte dargestellt werden; diese sind in der Peripherie des Gesichtsfeldes noch wahrzunehmen, wogegen visusangepaßte Optotypen unter Umständen nicht mehr benennbar sind. Dank der Erfindung kann die echte Grenze des Exkursionsvermögens mit anderen Sehzeichen exakter ermittelt werden, deren Benennung in der Peripherie nicht mehr möglich ist und die sich auch per Zufallsgenerator ändern können, so daß sie der Proband nicht aus der Erinnerung identifizieren kann.

Indem laut Anspruch 13 und Anspruch 29 bei einer Untersuchung an einer Tangententafel die objektive Stellung eines Augapfels oder beider Augäpfel des Probanden überwachbar ist, kann man die Ermittlung der subjektiven Schielwinkel problemlos in ein und derselben Messung damit kombinieren. So lassen sich Hornhaut-Reflexbilder, die z.B. mit einer Infrarotkamera aufgenommen wurden, mit den erfindungsgemäß gewonnenen Daten ohne weiteres in Relation setzen. Durch Aufnahme der objektiven Stellung beider Augäpfel zueinander z.B. mittels der Hornhaut-Reflexbilder ist die dreidimensionale Feststellung von objektiven Zyklo-Deviationen ebenfalls möglich. Mit der neuen Anlage können Messungen wahlweise bei bewegtem oder bei festgelegtem Kopf durchgeführt werden, und es sind sowohl Hess- als auch Helmholtz-Koordinaten erfaß- und speicherbar. In der klinischen Schielbehandlung gibt es eine große Anzahl verschiedener Methoden, die aber manuell praktiziert werden müssen, was häufig extrem viel Zeit und Geduld sowohl des Fachpersonals als auch der Patienten erfordert. Deutliche Zeitersparnis und mithin kostengünstigere, auch angenehmere Untersuchungen erzielt man erfindungsgemäß, indem ein einziges Untersuchungssystem unter gleichen Untersuchungsbedingungen alle motorischen und sensorischen Augenstörungen automatisch erfaßbar macht. So lassen sich einbeziehen: die Bestimmung des Winkels Kappa, harmonisch und disharmonisch anomale Korrespondenzen, foveal-periphere Korrespondenz-Unterschiede, Kampimetrie der Hemmungsskotome, Blickzielbewegungen und okuläre Nystagmusdiagnose.

Anspruch 14 sieht vor, daß die Überprüfung von Einstellbewegungen der Augen des Probanden durch getrennte und alternierende Darbietung von Fixierobjekten erfolgt, wobei die Seheindrücke auf der virtuellen Tangententafel solange bewegt oder neu positioniert werden, bis keine Augen-Einstellbewegung mehr stattfindet. Dabei entspricht die relative Lage der dargebotenen Seheindrücke des rechten und des linken Auges zueinander dem objektiven Schielwinkel. Dies gestattet die automatische Erfassung aller Schielwinkel an einer Tangententafel, und zwar auch von Probanden mit einer anomalen Netzhautkorrespondenz oder mit einer Exklusionszone. Laut Anspruch 30 dient zur blickgetrennten Darbietung von Fixierobjekten namentlich ein frequenzsteuerbarer Shutter, mit dem alternierend oder wahlweise ein linker und ein rechter Sichtkanal bzw. ein linkes und ein rechtes Teilbild okkludierbar ist. Wiederum kommt man dem natürlichen Sehen sehr nahe, da - anders als bei farblicher Trennung - kein Fehler durch Akkommodation auftritt. Nun können an Tangententafeln auch Binokulartests durchgeführt werden; auch die Grade des Binokularsehens (Simultansehen, Fusion, Fusionsbreite und Stereosehen) lassen sich testen.

Gemäß Anspruch 15 werden Einstellbewegungen eines Auges oder beider Augen automatisch erfaßt und die an der Tangententafel gemessenen simultanen oder alternierenden objektiven (Horizontal-, Vertikal-, Zyklo-)Schielwinkel den subjektiven (Horizontal-, Vertikal-, Zyklo-)Deviationen zugeordnet. Das bewirkt einen beachtlichen Fortschritt, denn herkömmlich konnten nur die subjektiven Schielwinkel jener Minderheit von Patienten untersucht werden, die eine normale Netzhautkorrespondenz und gleichzeitig Doppelbilder haben.

Eine weitere Ausgestaltung ergibt sich aus den Merkmalen des Anspruchs 16, wonach sich ein Verfahren der eingangs genannten Art dadurch auszeichnet, daß bei freisichtigen Untersuchungen rechnerisch für jedes Auge getrennt ein Koordinatensystem auf die Pupillenmitte bezogen wird und daß die Ursprünge jedes Koordinatensystems wahlweise um den Augenabstand des Probanden zueinander versetzt oder koinzident auf der Mitte zwischen den Augen (Nasenwurzel) vereinigt werden. Man kann daher in herkömmlicher Weise ein "Zyklopenauge" als fiktives Sehzentrum benutzen oder die Untersuchungen an Tangententafeln auf neuartige Weise realoptisch präzisieren.

Eine andere wichtige Ausgestaltung der Erfindung besteht nach Anspruch 17 und Anspruch 31 darin, daß zur Feststellung der Kopfhaltung und ihrer Änderungen am Probandenkopf Trägerstücke oder -flächen für Meßwert-Geber und/oder -Sensoren eines Kontrollsystems vorhanden bzw. anbringbar sind, um Abweichungen zwischen Soll- und Ist-Kopfhaltung eines Probanden während einer Untersuchung an einer Tangententafel dem Untersucher automatisch anzuzeigen. Geeignete Gebern bzw. Sensoren sind z.B. Infrarot-Sender und/oder -Empfänger, die am frei beweglichen oder fixierten Kopf des Probanden anbringbar sind, alle Kopf-Positionen sowie -Bewegungen einschließlich -Neigungen quantitativ differenziert melden und die Untersuchung bei Erreichen eines vorgegebenen Unterschieds zwischen Soll- und Ist-Parameterwert unterbrechen, zumindest bis die Korrektur einer Kopf-Fehlstellung erfolgt. Dazu können beispielsweise zwei oder mehr virtuelle Objekte passend positioniert werden, die mit Daten der Kopfposition des Probanden versorgt sind und eine gegebene virtuelle Zone nicht verlassen dürfen. Besonders günstig ist dabei, daß Toleranzgrenzen variabel einstellbar sind.

Eine praktische Ausführungsform eines Kopfgerätes weist laut Anspruch 32 ein stirnseitig ansetzendes Band, einen Klemmstreifen o.dgl. auf, an oder nahe dessen hinterem Ende ein Querstreifen zu den Ohrenbereichen führt, so daß weite Teile der Schädeloberfläche nicht verdeckt sind. Die Vorrichtung ist entsprechend leicht und unterscheidet sich somit vorteilhaft von herkömmlichen Helmen oder Hauben. Dem Probanden sind ungezwungene Kopfbewegungen möglich, auch wenn die Geber bzw. Sensoren an einen Computer oder dessen Peripheriegeräte angeschlossen sind. Man kann Untersuchungen des BES-Feldes, des Fusionsblickfeldes sowie der Gebrauchsblickrichtung durchführen und die Kopfzwangshaltung bestimmen.

Gemäß Anspruch 18 und Anspruch 33 ist ferner eine integrierte akustische Begleitsteuerung möglich, d.h. eine Vorgabe an die Anlage seitens einer Person z.B. unter Verwendung von Mikrofonen, Lauthöreinrichtungen usw., und insbesondere auch eine in Abhängigkeit von der laufenden Untersuchung synthetisch erzeugte und/oder digitalisierte Sprachführung, d.h. eine Anweisung der Anlage an Personen, nämlich für den Probanden und/oder den Bediener.

Zur besonders bequemen und exakten Beobachtung sowie grafischen und/oder numerischen Registrierung der Kopfhaltung sieht Anspruch 34 Videografie-Einrichtungen vor, insbesondere Infrarot-Kameras und -Strahler.

Wenn entsprechend Anspruch 19 und Anspruch 35 ein separater Bedienerbildschirm zur Darstellung eines Untersucherbildes zusätzlich zu dem TangentenTafelbild, welches der Proband sieht, verwendet wird, ergeben sich schon während der Untersuchung bequem ergänzende Möglichkeiten der Diagnosefindung für den Untersucher. Er vermag den Ablauf so auf einfache Weise zu gestalten. Während der Proband ununterbrochen und ungestört die Tangententafel betrachtet, kann der Bediener gleichzeitig ohne irgendwelche Umschaltung den separaten Schirm beobachten und nach Bedarf per Sprache oder sonstige Signale dem Probanden Hinweise oder Anweisungen geben.

Im Einklang mit Anspruch 20 und Anspruch 36 können die an einer Tangententafel dargestellten Seheindrücke wahlweise oder zusätzlich von einer optoelektronischen Einrichtung abgetastet und sogleich abrufbar gespeichert werden. Die im Rechner vorhandenen Funktionen in Form von Gleichungen oder als Menge von Stützpunkten lassen sich dabei sofort in Beziehung zu den Abtastwerten (Positionen und Winkeln) setzen, so daß unmittelbar eine differenzierte Beobachtung und Auswertung erzielt wird. Durch Datenablage wird auch hierbei das Protokollieren und die Führung von Patientenakten außerordentlich erleichtert.

Weitere Merkmale, Einzelheiten und Vorteile ergeben sich aus dem Wortlaut der Ansprüche sowie aus der folgenden Erläuterung von Ausführungsbeispielen anhand der Zeichnung. Darin zeigen:
- Fig. 1: eine vereinfachte Schrägansicht einer Seh-Untersuchungs-Anlage in einem Hüllraum,
- Fig. 2: eine schematisierte Seitenansicht einer Projektionseinrichtung,
- Fig.3: eine schematisierte Seitenansicht einer anderen Projektionseinrichtung,
- Fig. 4: eine Draufsicht auf ein Handsteuergerät,
- Fig.5: eine Seitenansicht eines an einem Kopf angebrachten Kontrollsystems,
- Fig. 6a: eine Rückansicht eines Kontrollsystems entsprechend Fig. 5,
- Fig. 6b: eine Draufsicht auf das Kontrollsystem von Fig. 6a,
- Fig. 7a: eine Seitenansicht,
- Fig. 7b: eine Rückansicht sowie eines anderen an einem Kopf
- Fig. 7c: eine Draufsicht angebrachten Kontrollsystems,
- Fig. 8: eine Schrägansicht noch eines weiteren Kontrollsystems,
- Fig. 8a: eine Teil-Draufsicht auf die Mitte eines Oberteils des Kontrollsystems von Fig. 8,
- Fig. 9a: eine schematisierte Seitenansicht der Anordnung eines Augenvorsatzes,
- Fig. 9b: eine Vorderansicht des Augenvorsatzes von Fig. 9a,
- Fig. 10a: eine schematisierte Draufsicht und
- Fig. 10b: eine Seitenansicht einer Anordnung zur Hornhautreflex-Erzeugung,
- Fig. 10c und 10d: je eine zunehmend vergrößerte Augansicht mit Hornhautreflexen,
- Fig. 11: ein Bildschema der Untersuchungsverfahrens mit virtuellen Tangententafeln,
- Fig. 12a: eine Schema-Schrägansicht einer Laserprojektions-Anordnung und
- Fig. 12b: ein schematisiertes Teilbild mit dem Kreis Xllb in Fig. 12a zugeordneten Funktionselementen.

Die folgenden Beispiele einer erfindungsgemäßen Anlage mit zugehörigen Vorrichtungen betreffen Vorzugs-Varianten; welche davon zum praktischen Einsatz kommt, entscheidet der Facharzt nicht zuletzt anhand der örtlichen Gegebenheiten eines Untersuchungsraumes und seiner Anforderungen.

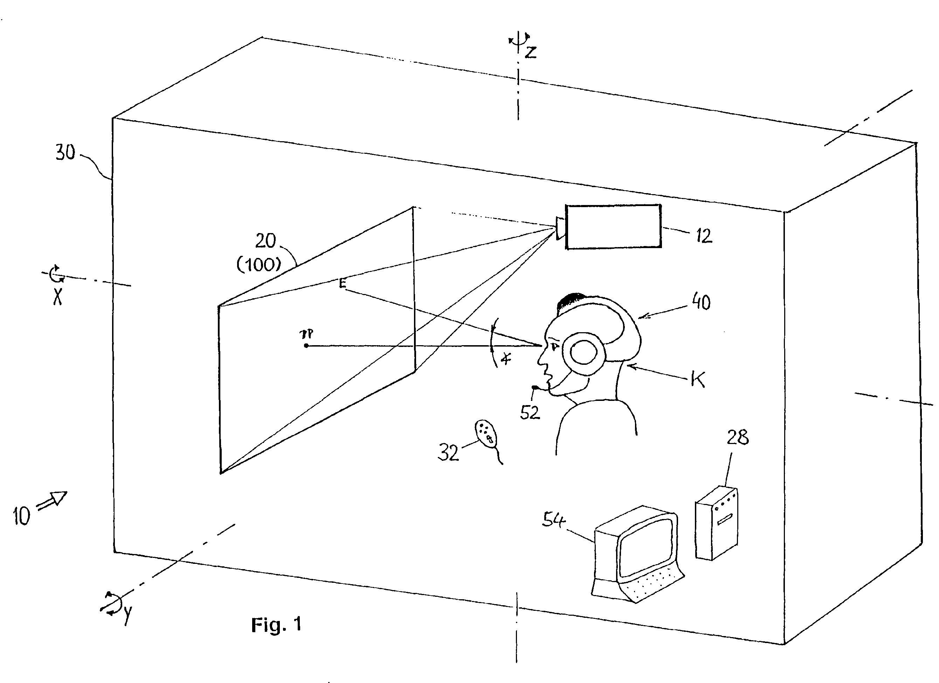

Man erkennt in Fig. 1 eine allgemein mit 10 bezeichnete Seh-Untersuchungs-Anlage. Sie weist in einem Hüllraum 30, dem Raum-Koordinatenachsen X, Y, Z zugeordnet sind, einen Projektor 12 auf, der mittels Aufprojektion eine virtuelle Tangententafel auf einer Projektionsfläche 20 erzeugt. Vor dieser befindet sich ein Proband, an dessen Kopf K ein Kontrollsystem 40 sowie ein Mikrofon 52 angebracht ist. Mit einem Handgerät 32 kann der Proband anzeigen, was er sieht, und er kann die Seheindrücke beeinflussen. Untersucht wird hier die Abweichung der Blickrichtung eines Auges zwischen der Primärposition PP und einem Fixierobjekt bzw. Seheindruck E, beispielsweise einem E-Haken als Sehzeichen (Optotype).

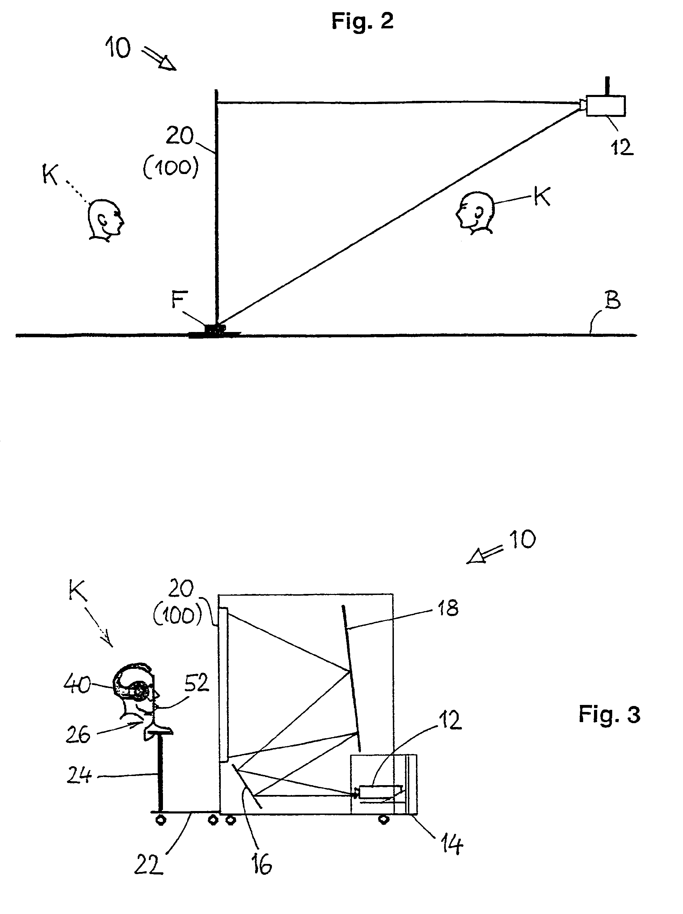

Fig. 2 veranschaulicht schematisch den Projektor 12 mit einem Strahlengang zu der Darstellungsfläche 20, die z.B. als bewegliche Vertikalwand mit einem Fuß F auf dem Boden B eines Raumes steht. Ein Proband kann eine projizierte virtuelle Tangententafel von der Projektorseite betrachten, d.h. in Aufprojektion, oder - was gestrichelt angedeutet ist - von der Gegenseite her, also in Rückprojektion.

Im Beispiel der Fig. 3 befindet sich das Projektionssystem 12 der Anlage 10 im Inneren eines Gehäuses 14, das als integrale Rückprojektionsbox ausgebildet sein oder auch rückseitig offen sein kann, damit bestimmte Projektortypen zur Anpassung an unterschiedliche Projektionsabstände nach hinten herausragen können. Für die Darstellung der virtuellen Tangententafel sind zwei Umlenkspiegel 16, 18 in einstellbarer Winkelposition so angeordnet, daß auf der Darstellungsfläche 20 die gewünschte Bildgröße der Tangententafel erreicht wird. Deren Format richtet sich gemäß rechnerischen Zusammenhängen nach dem vom Facharzt vorgegebenen Betrachterabstand. Der Proband befindet sich auf der dem Projektor 12 gegenüberliegenden Seite vor der Projektionsfläche 20, die er freisichtig oder durch ein Bildtrennungssystem von solcher Ausbildung betrachten kann, daß keine nennenswerte Beeinträchtigung der vorhandenen Seheindrücke gegeben ist. Diese nimmt er - bis auf eine minimale prismatische Nebenwirkung von Farbfiltergläsern - in ihren tatsächlichen Positionen auf der Darstellungsfläche 20 wahr.

Wird mit der Anlage 10 Koordimetrie durchgeführt, so fixiert man den Kopf K des Probanden in definierter Position vor der Darstellungsfläche 20 in einer Kopfstütze 26, die auf einem Ständer 24 eines Fahrgestells 22 angeordnet sein kann. Die übrigen Komponenten der Anlage 10 sind gleichfalls verfahrbar. Schematisch sind ein Kopfkontrollsystem 40 und ein Mikrofon 52 angedeutet, das zur sprachlichen Beeinflussung des Untersuchungsablaufs dienen kann. Falls nicht benötigt, kann das Kontrollsystem 40 z.B. per Fernbedienung oder Software deaktiviert werden.

Der große Vorteil der in Fig. 3 veranschaulichten Rückprojektion besteht darin, daß sehr geringe Betrachterabstände möglich sind, ohne daß der Proband den Projektionsstrahlengang durch Schattenwurf stört. Im Falle der Aufprojektion (Fig. 1) befindet sich das Projektionssystem 12 - vorzugsweise nahe der Decke des Untersuchungsraumes - auf der gleichen Seite wie der Proband. Kann für eine ausreichend große virtuelle Tangententafel eine kurze Projektionsdistanz gewählt werden (z.B. dank kleiner Brennweite eines Objektivs), so läßt sich der Projektor 12 auch am Boden vor dem Probanden oder unter diesem Probanden anordnen, z.B. in einem Stuhl integriert. Die Aufprojektion ist besonders gut geeignet, wenn als Tangententafel eine Harms-Wand virtuell erzeugt wird, die wegen des erforderlichen größeren Betrachterabstands auch eine große Darstellungsfläche 20 benötigt.

Soll auf Wunsch des Arztes die Projektion auf eine körperlich vorhandene Tangententafel erfolgen, so ist diese Kombination mit der virtuellen Tangententafel bei Aufprojektion ebenfalls möglich. Die Untersuchungen können dann auch an einer realen Tangententafel schneller durchgeführt und die Ergebnisse an ihr automatisch erfaßt werden, wozu das rechnerisch vorliegende Koordinatensystem der virtuellen Tangententafel mit dem physikalisch festen Koordinatensystem der realen Tangententafel (z.B. der Harms-Wand) in Deckung gebracht wird.

Die Projektionsfläche 20 kann an der Wand schwenkbar oder an der Decke wie eine Leinwand ausrollbar montiert, aber auch fest im Untersuchungsraum montiert oder versetzbar aufgestellt sein. Wird sie wie ein Raumteiler freistehend angeordnet (Fig. 2), so ist die Untersuchung und die Betrachtung der virtuellen Tangententafel durch den Probanden nach Bedarf von der einen oder der anderen Seite her möglich. Dann können bei geringen Betrachterabständen des Probanden einerseits Untersuchungen mit Rückprojektion und andererseits bei größerem Betrachterabstand mit Aufprojektion durchgeführt werden. Die virtuellen Tangententafeln lassen sich bei Rückprojektion auch durch eine Software gespiegelt dargestellen. Infolgedessen kann beispielsweise nacheinander mit Koordimetrie rückseitig in geringem Betrachterabstand untersucht werden, während von der Aufprojektionsseite die gleiche oder eine andere virtuelle Tangententafel (z.B. eine Harms-Wand) in größerem Abstand dargeboten wird, was bisweilen vom Facharzt gewünscht wird.

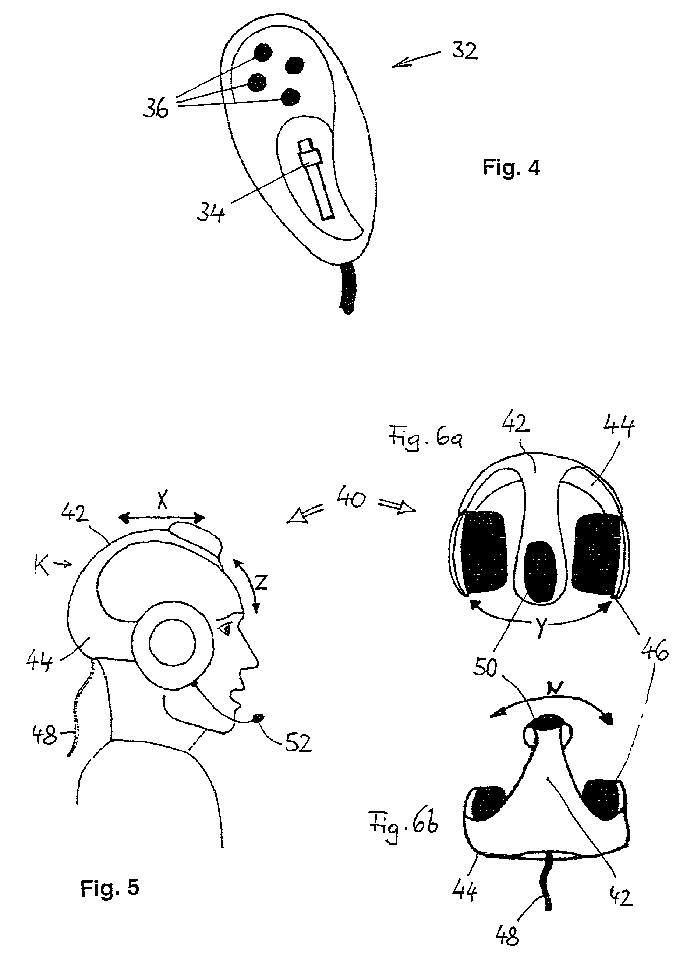

Fig. 4 stellt ein Handgerät 32 dar, das als typische Fernbedienung gestaltet sein und mit dem ein Proband während der Untersuchung Signale abgeben kann. Dazu hat das Gerät 10 einen Schieberegler 34 und eine Anzahl von Einstellknöpfen 36, etwa um verschiedene Lichtzeiger zu betätigen oder Seheindrücke zu steuern.

Ein Kopfkontrollsystem 40 ist in Fig. 5 sowie Fig. 6a und 6b gezeigt. Es hat oben einen vorzugsweise schmalen Bügel oder Klemmstreifen 42, der sich hinten bzw. unten mit einem Querstreifen oder Träger 44 für Hörmuscheln 46 sowie für einen Kabelanschluß 48 fortsetzt. Seitlich ist ein Mikrofon 52 angeordnet.

Das Kontrollsystem 40 ist mit Gebern und/oder Sensoren bestückt, die bevorzugt als Infrarot-Sender/Empfänger 50 ausgebildet sein können und es erlauben, Änderungen der Kopfhaltung in verschiedenen Richtungen und Winkeln fortlaufend zu beobachten und zu registrieren, etwa mittels (nicht gezeichnetem) Bildschirm und Datenspeicher. Beispiele von Änderungsbereichen sind mit Doppelpfeilen X (Fig. 6a) für die horizontale Schwenkbewegung, Y für die horizontale Vor-/Rück-Bewegung sowie Z für die Höhenänderung (Fig. 5) und N für die Seitenneigung (Fig. 6b) des Kopfes K angedeutet.

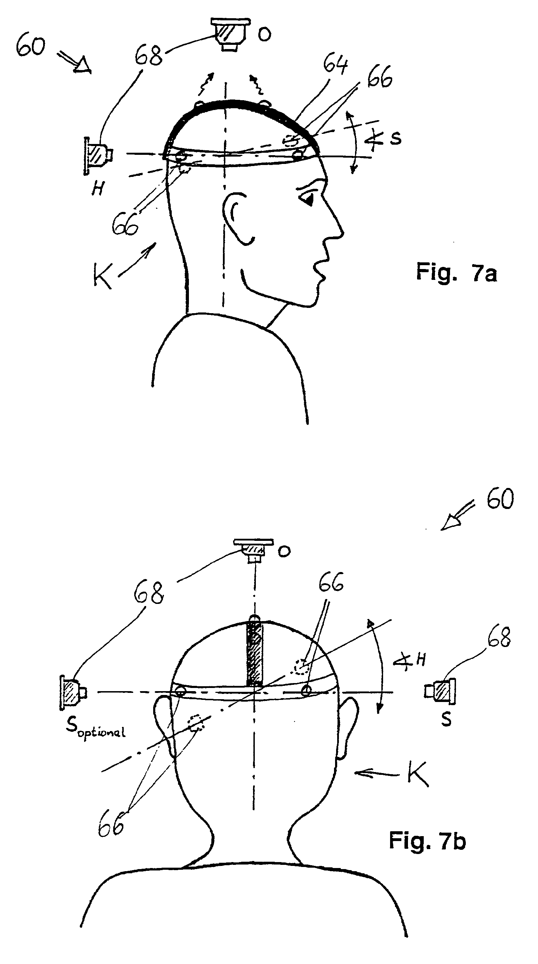

Ein anderes Kopfkontrollsystem 60 ist aus Fig. 7a bis 7c ersichtlich. Es hat ein Stirnband 62, das mit einem Mittelband oder -bügel 64 den Kopf K schädelseitig umschließt. An diesen schmalen Riemen oder Bändern 62, 64 sind in ausgewählten Winkel- bzw. Umfangsabständen Geber und/oder Sensoren paarig angeordnet. Dies können beispielsweise Ohmsche Widerstände, IR-strahlende Dioden usw. sein; bevorzugt sind jedoch Infrarot-Strahler bzw. IR-Fotozellen 66 vorgesehen. Durch je zwei solcher Elemente ist ein Geradenverlauf definiert. Er gestattet es, Kopfachsen-Abweichungswinkel zu messen, die von Video- oder IR-Kameras 68 oben (O), an der Seite (S) und hinten (H) quantitativ erfaßt und zum Beispiel im (nicht dargestellten) Rechner als Zahlenwerte oder auch grafisch gespeichert werden können. Man erkennt, daß auf diese Weise die ungezwungene Kopfhaltung ebenso wie eine eventuelle Kopfzwangshaltung des Probanden zu jedem Zeitpunkt einer Untersuchung beobachtet, exakt registriert und mithin sauber protokolliert werden kann. Jede solche Beobachtung läßt sich auch auf einem separaten Untersucher-Bildschirm 54 (Fig. 1) verfolgen.

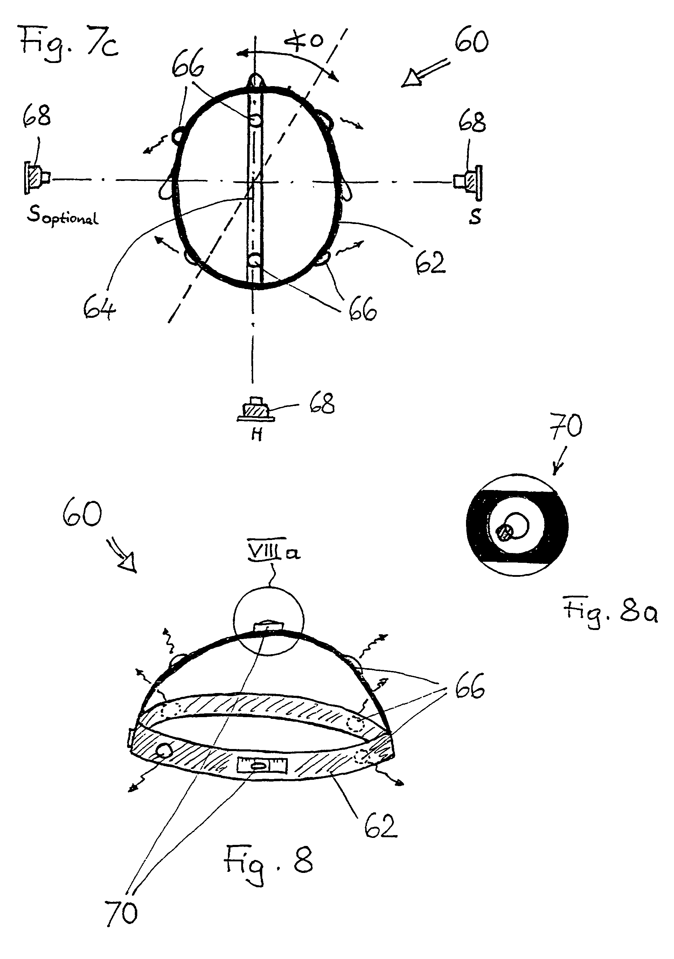

Noch eine andere Variante eines Kopfkontrollsystems geht aus Fig. 8 und 8a hervor. Das System 60 hat hierbei wiederum ein Stirnband 62 sowie einen Mittelstreifen oder -bügel 64 und paarig angeordnete Geber/Sensoren 66. Zusätzlich sind jeweils mittig Neigungssensoren, namentlich kleine Wasserwaagen oder Libellen 70 vorhanden. Man hat damit einfache Nivelliereinrichtungen, die eine korrekte Positionierung von (nicht gezeichneten) Trägerstücken für die Geber/Sensoren 66 erleichtern und auch eine Kopf-Sollstellung festzulegen gestatten. Diese Nivelliermittel können auch mit Skalen und Winkeleinteilungen versehen sein, die zumindest eine Grobmessung der Kopfhaltung ermöglichen.

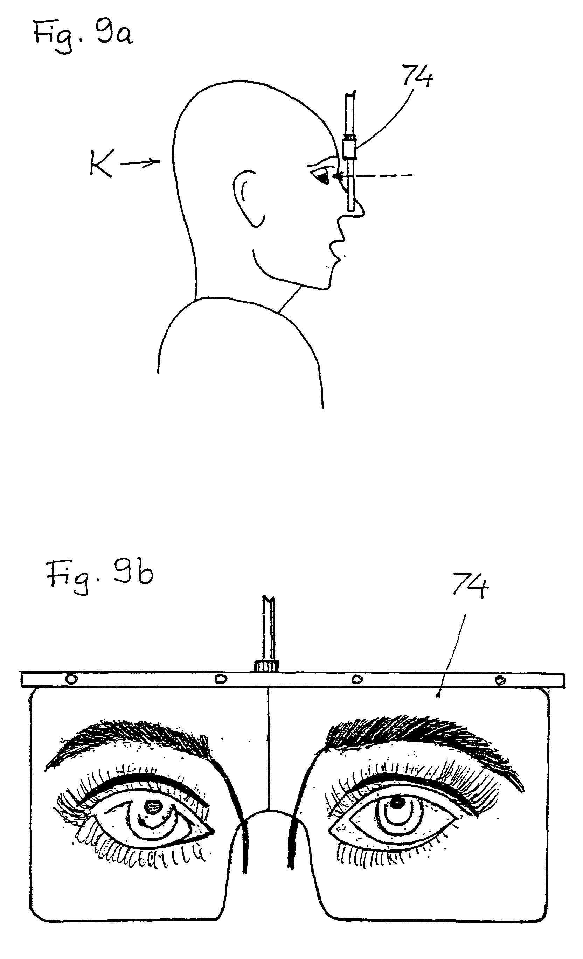

Fig. 9a und 9b veranschaulichen beispielhaft einen Vorsatz 74 zur Bildtrennung. Derartige an sich bekannte Einrichtungen dienen dazu, einen linken Bildkanal von einem rechten Bildkanal zu trennen, z.B. durch ein- oder beidseitig eingesetzte Farbfilter. Vielfach wird nur ein Dunkelrot-Glas vor dem fixierende Auge benutzt, doch lassen sich linkes und rechtes Teilbild auch durch unterschiedliche Farbvorsätze trennen. Gleichartig angeordnete (nicht gezeichnete) Shutter können LCD-Anordnungen aufweisen, d.h. Liquid Crystal Displays, die wechselnd durchlässig (klar) und undurchlässig (schwarz) gesteuert werden können. Das kann durch Einzel-Ansteuerung, aber nach Bedarf auch nieder- oder hochfrequent geschehen.

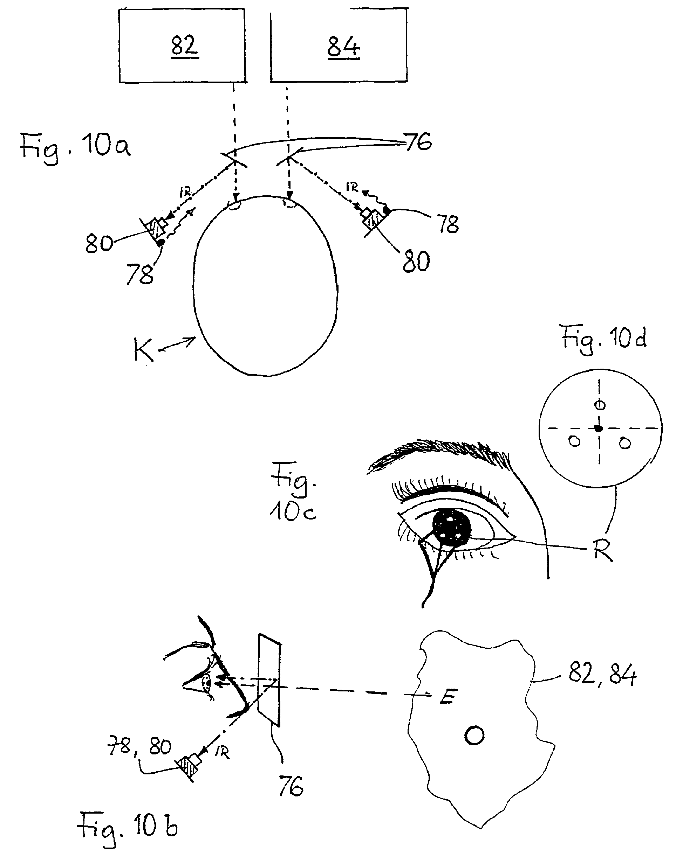

Eine Anordnung zum Erzeugen von Hornhaut-Reflexbildern (Fig. 10c und 10d) geht aus Fig. 10a und 10b hervor. Hieraus ersieht man, daß vor den Augen eines Probanden Reflexspiegel 76 angeordnet sind, die als sog. warm mirrors zwar für sichtbares Licht durchlässig sind, Infrarotstrahlung jedoch reflektieren. Beiderseits des Kopfes K ist jeweils ein IR-Strahler 78 und eine IR-Kamera 80 so angebracht, daß bei gerade nach vorn - nämlich auf Sehzeichen-Tafeln - gerichtetem Blick des Probanden die Kameras 80 jeweils Reflexbilder der angestrahlten Hornhautin empfangen. Beispiele solcher Aufnahmen sind in Fig. 10c und 10d mit zunehmnder Vergrößerung dargestellt.

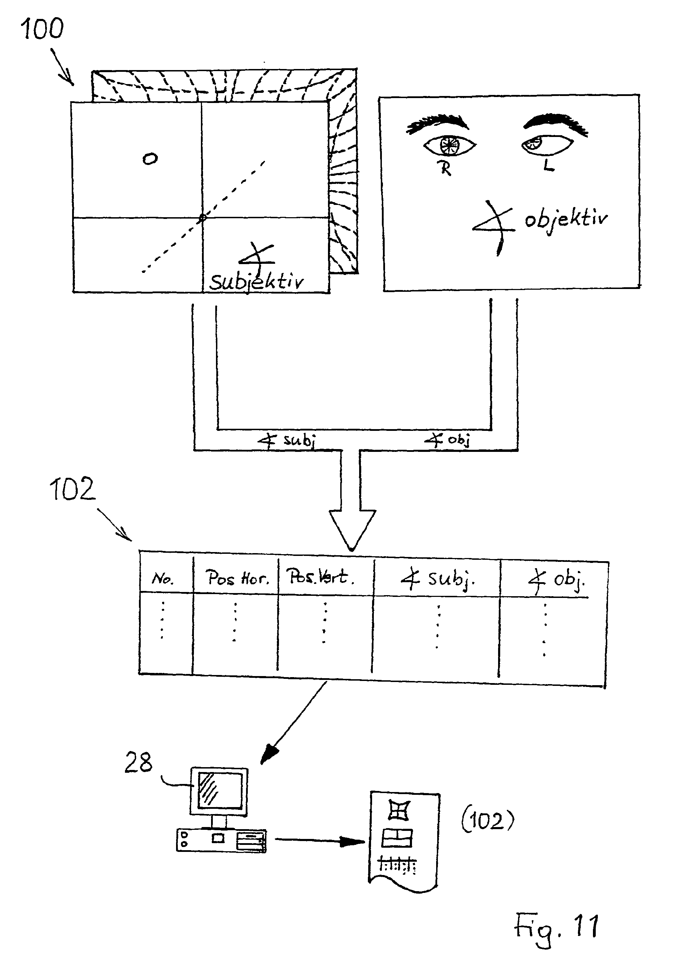

Fig. 11 vermittelt eine Übersicht über die Methodik von Untersuchungen nach der Erfindung. Links oben ist eine Tangententafel 100 zu sehen, die gegenständlich, virtuell oder kombiniert körperlich-virtuell sein kann. Im Vordergrund ist schematisch eine herkömmliche Tangententafel (Harms-Wand oder Maddox-Kreuz) vor einem wahlweise anwendbaren Koordimetrieschirm gezeichnet. Angedeutet sind subjektive Deviationen gemäß Zeige-Angaben des Probanden. Rechts oben in Fig. 11 ist schematisch veranschaulicht, wie der objektive Schielwinkel entweder über Shutter und Einstellbewegungen oder mittels IR-Kameras ermittelt wird. Die Messung erfolgt gleichzeitig und bei gleicher Blickrichtung, d.h. in einem Gang unter völlig gleichen Bedingungen, und die Ergebnisse werden zusammengeführt sowie aufgezeichnet. In der Mitte von Fig. 11 ist ein Beispiel eines Protokolls 102 dargestellt, das über den REchner 28 in wählbarem Format erstellt werden kann, wie unten angedeutet ist.

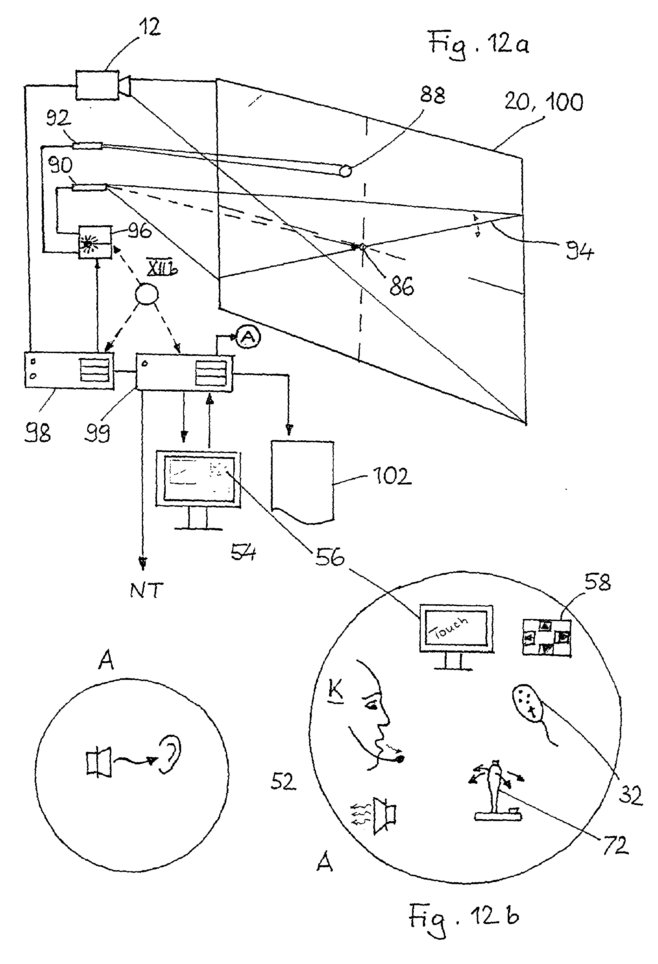

Aus Fig. 12a sieht man, wie eine körperliche (und/oder virtuelle) Tangententafel 100 optoelektronisch abgetastet werden kann. Der Projektor 12 wirft das gewünschte Muster auf die Tafel 100, auf der ein zentrales Fixierlicht 86 z.B. rot und ein weiteres Objektlicht 88 z.B. grün erscheinen kann. Diese Lichter 86, 88 werden von einem roten Laserprojektor 90 bzw. einem grünen Laserprojektor 92 ausgestrahlt. Der Rotlicht-Projektor 90 gibt auch einen Zyklostrahl 94 ab. Ein Sehzeichengeber 96 steuert die Projektoren 90, 92 und wird seinerseits über einen Geber-Rechner 98 gesteuert, der mit dem Projektor 12 und außerdem mit einem Steuer-Rechner 99 verbunden ist. Letzterer kann an ein Netzwerk NT in üblicher LAN- oder WAN-Technik angeschlossen sein, je nach örtlicher oder überregionaler Netz-Anbindung. Die Bedienung der Steuereinheiten 96, 98, 99 kann über Funktionselemente erfolgen, die entsprechend dem Kreis Xllb in Fig. 12b schematisch angedeutet sind. Dazu gehören ein Mirofon 52 am Kopf K des Probanden, Audiosystem A, ein Steuerknüppel oder Joystick 72, ein Handgerät 32, ein Tastenfeld 58 sowie ein Touchscreen 56, der dem Bediener-Bildschirm 54 zugeordnet ist. Über den Steuer-Rechner 99 können Ausdrucke als Protokoll 102 ausgegeben werden.

Die Erfindung ist nicht auf die beschriebenen Ausführungsformen beschränkt; vielmehr sind zahlreiche Abwandlungen möglich. So müssen die genannten Geber/Sensoren 66 nicht an Riemen, Bändern, Streifen u.dgl. befestigt sein; es eignen sich auch Saugnäpfe, Haftpunkte kleine Klettflächen u.dgl., die einzeln an ausgewählten Kopfstellen angebracht werden.

Man erkennt jedoch, daß sich die Erfindung mit der automatischen Erfassung, Speicherung und Auswertung von Meßwerten bei Seh-Untersuchungen in einer Anlage 10 befaßt, welche die Erzeugung und Steuerung von Seheindrücken E sowie die interaktive Steuerung des Untersuchungsablaufes ermöglicht. Auf einer Tangententafel 100 durch ein Projektionssystem 12 entworfen. Ein Bildtrennungssystem 74 und Einrichtung 76...80 zum Ermitteln der objektiven Stellungen der Augäpfel sowie ein Kontrollsystem 40, 60 zur Überwachung der Kopfhaltung gewährleisten automatische Messung und Registrierung der Beobachtungen. Die Darstellungsfläche kann interaktiv beeinflußt und per Videografie oder Fotografie abgetastet werden.

Sämtliche aus den Ansprüchen, der Beschreibung und der Zeichnung hervorgehenden Merkmale und Vorteile, einschließlich räumlicher Anordnungen, konstruktiver Einzelheiten und Verfahrensschritten, können sowohl für sich als auch in den verschiedensten Kombinationen erfindungswesentlich sein.

### Bezugszeichenliste

- A: Audiosystem
- B: Boden
- E: Seheindruck [Fixierobjekt]
- F: Fuß (eines Raumteilers)
- H: hintere Kamera-Position
- K: Kopf (des Probanden)
- N: Seitenneigung
- NT: Network-Anschluß
- O: obere Kamera-Position
- PP: Primärposition
- R: Reflexbilder
- S: seitliche Kamera-Position
- w: Blickwinkel
- X, Y, Z: Raum-Koordinatenachsen

- 10: Anlage
- 12: Projektor
- 14: Gehäuse
- 16: erster Umlenkspiegel
- 18: zweiter Umlenkspiegel
- 20: Projektionsfläche
- 22: Fahrgestell
- 24: Ständer
- 26: Kopfstütze
- 28: Rechner
- 30: Hüllraum
- 32: Handgerät
- 34: Schieberegler
- 36: Einstellknöpfe
- 38: Kabel
- 40: (Kopf-) Kontrollsystem
- 42: Bügel / Klemmstreifen
- 44: Querstreifen / Träger
- 46: Hörmuscheln
- 48: Kabel
- 50: Sender / Empfänger
- 52: Mikrofon
- 54: Bediener-Bildschirm
- 56: Touchscreen
- 58: Tastenfeld
- 60: (Kopf-) Kontrollsystem
- 62: Stirnband
- 64: Mittelband / -bügel
- 66: (IR-) Geber / Sensoren
- 68: (IR-) Kameras
- 70: Libelle
- 72: Joystick
- 74: (Filter-)Vorsatz
- 76: Reflexspiegel
- 78: IR-Strahler
- 80: IR-Kamera
- 82: linke Sehzeichen-Tafel
- 84: rechte Sehzeichentafel
- 86: (rotes) Fixierlicht
- 88: (grünes) Objektlicht
- 90: roter Laserprojektor
- 92: grüner Laserprojektor
- 94: (roter) Zyklostrahl
- 96: Sehzeichengeber
- 98: Geber-Rechner
- 99: Steuer-Rechner
- 100: Tangententafel
- 102: Protokoll

## Patentansprüche

1. Verfahren zum Durchführen von Seh- Untersuchungen, insbesondere bei Probanden-Sehstörungen wie Strabismus, unter Verwendung von Projektions-Einrichtungen, Seheindruck-Gebern, Fixier- und Zeigelicht, Tangententafeln sowie elektronischen und computerunterstützten Meß- und Auswertemitteln, mit Überwachung der Kopf- und/oder Augapfel-Position, **dadurch gekennzeichnet, daß** auf einer Darstellungsfläche rechnergesteuert eine virtuelle Tangententafel erzeugt wird, zu der koordinatenbezogene Seheindrücke freisichtig dargeboten werden.

2. Verfahren nach Anspruch 1, **dadurch gekennzeichnet, daß** die Darstellungsfläche mit der virtuellen Tangententafel in einem den Probanden umgebenden Hüllraum angeordnet wird.

3. Verfahren nach Anspruch 1 oder 2, **dadurch gekennzeichnet, daß** während der Untersuchung Seheindrücke und Verfahrensschritte vom Untersucher und/oder vom Probanden interaktiv beeinflußbar sind, namentlich durch elektronische Hilfsmittel mit Fernbedienung, durch Touchscreen, Tastenfeld, Steuerknüppel (Joystick), Mikrofon u.dgl.

4. Verfahren nach einem der Ansprüche 1 bis 3, **dadurch gekennzeichnet, daß** man die virtuelle Tangententafel und/oder die Seheindrücke auf einem Computermonitor, Großbildschirm o.dgl. erzeugt.

5. Verfahren nach einem der Ansprüche 1 bis 4, **dadurch gekennzeichnet, daß** Bilddaten eines Computers und/oder Videorecorders projiziert werden.

6. Verfahren nach einem der Ansprüche 1 bis 5, **dadurch gekennzeichnet, daß** man die virtuelle Tangententafel und/oder die Seheindrücke mit Hilfe eines Projektionssystems durch Aufprojektion und/oder Rückprojektion erzeugt.

7. Verfahren nach einem der Ansprüche 2 bis 6, **dadurch gekennzeichnet, daß** zum Darbieten und/oder Positionieren von Seheindrücken auf einer Tangententafel innerhalb des Hüllraumes zumindest ein Laser-Projektor verwendet wird, jedoch nicht an einer Kopfstütze oder als deren Bestandteil.

8. Verfahren nach einem der Ansprüche 1 bis 7, **dadurch gekennzeichnet, daß** man das Tangententafel-Koordinatensystem gemäß veränderlichen Parametern wie Ursprungsversetzung, Betrachter-Abstand, Probanden-Kopfhaltung usw. berechnet, insbesondere in einer Rechnereinheit unter Verwendung von Algorithmen, Funktionsgleichungen u.dgl.

9. Verfahren nach einem der Ansprüche 1 bis 8, **dadurch gekennzeichnet, daß** man das Koordinatensystem der virtuellen Tangententafel mit einem Hintergrundbild und/oder mit einer gegenständlichen Tangententafel kombiniert.

10. Verfahren nach einem der Ansprüche 1 bis 9, **dadurch gekennzeichnet, daß** man in Verbindung mit der virtuellen Tangententafel beliebige Seheindrücke verwendet.

11. Verfahren nach einem der Ansprüche 1 bis 10, **dadurch gekennzeichnet, daß** man die Meßergebnisse unter grafischer und/oder numerischer Aufzeichnung automatisch und ausdruckbar protokolliert.

12. Verfahren nach einem der Ansprüche 1 bis 11, **dadurch gekennzeichnet, daß** mit der Untersuchung an einer virtuellen Tangententafel andere blickrichtungsabhängige Sehprüfungen z.B. des monokularen oder binokularen Sehens, Visustest usw. kombiniert werden, wobei die Sehzeichen über das gesamte Blick- und Sichtfeld hinweg bewegbar sind.

13. Verfahren nach einem der Ansprüche 1 bis 12, **dadurch gekennzeichnet, daß** bei einer Untersuchung an einer Tangententafel die objektive Stellung eines Augapfels oder beider Augäpfel des Probanden überwacht und innerhalb ein und derselben Messung seinen subjektiven Seheindrücken zugeordnet wird, z.B. den subjektiven Schielwinkeln.

14. Verfahren nach Anspruch 12 oder 13, **dadurch gekennzeichnet, daß** die Überprüfung von Einstellbewegungen der Augen des Probanden durch getrennte und alternierende Darbietung von Fixierobjekten erfolgt, wobei die Seheindrücke auf der virtuellen Tangententafel solange bewegt oder neu positioniert werden, bis keine Augen-Einstellbewegung mehr stattfindet.

15. Verfahren nach einem der Ansprüche 1 bis 14, **dadurch gekennzeichnet, daß** Einstellbewegungen eines Auges oder beider Augen automatisch erfaßt und die an der Tangententafel gemessenen simultanen oder alternierenden objektiven Schielwinkel den subjektiven Deviationen zugeordnet werden, wahlweise punktbezogen auf eines der Augen oder auf beide Augen oder auf die Mitte zwischen ihnen (Nasenwurzel).

16. Verfahren zum Durchführen von Seh- Untersuchungen, insbesondere bei Probanden-Sehstörungen wie Strabismus, unter Verwendung von Projektions-Einrichtungen, Seheindruck-Gebern, Fixier- und Zeigelicht, Tangententafeln sowie elektronischen und computerunterstützten Meß- und Auswertemitteln, mit Überwachung der Kopf- und/oder Augapfel-Position, nach einem der Ansprüche 1 bis 15, **dadurch gekennzeichnet, daß** bei freisichtigen Untersuchungen rechnerisch für jedes Auge getrennt ein Koordinatensystem auf die Pupillenmitte bezogen wird und daß die Ursprünge jedes Koordinatensystems wahlweise um den Augenabstand des Probanden zueinander versetzt oder koinzident auf der Mitte zwischen den Augen (Nasenwurzel) vereinigt werden.

17. Verfahren nach einem der Ansprüche 1 bis 15, **dadurch gekennzeichnet, daß** Abweichungen zwischen Soll- und Ist-Kopfhaltung eines Probanden während einer Untersuchung an einer Tangententafel dem Untersucher von einem Kontrollsystem automatisch angezeigt werden, wobei das am frei beweglichen oder fixierten Probandenkopf angeordnete Kontrollsystem insbesondere die Untersuchung unterbricht, sobald ein vorgegebener Parameter-Unterschied zwischen Soll- und Ist-Kopfhaltung erreicht oder überschritten wird.

18. Verfahren nach einem der Ansprüche 1 bis 16, **dadurch gekennzeichnet, daß** die Untersuchung von einer integrierten akustischen Steuerung begleitet wird, d.h. von einer Vorgabe an die Anlage seitens einer Person z.B. unter Verwendung von Mikrofonen, Lauthöreinrichtungen usw., und daß man eine in Abhängigkeit von der laufenden Untersuchung synthetisch erzeugte und/oder digitalisierte Sprachführung vorsieht, d.h. eine Anweisung der Anlage an Personen, nämlich für den Probanden und/oder den Bediener.

19. Verfahren nach einem der Ansprüche 1 bis 17, **dadurch gekennzeichnet, daß** ein separater Bedienerbildschirm zur Darstellung eines Untersucherbildes zusätzlich zu dem Tangententafelbild, welches der Proband sieht, verwendet wird.

20. Verfahren nach einem der Ansprüche 1 bis 10, **dadurch gekennzeichnet, daß** die an einer Tangententafel dargestellten Seheindrücke von einer optoelektronischen Einrichtung abgetastet und daß sie abrufbar gespeichert werden.

21. Anlage (10) zum Durchführen von Seh-Untersuchungen, insbesondere bei Probanden-Sehstörungen wie Strabismus, mit Projektions-Einrichtungen (12), Seheindruck-Gebern (96, 32), Fixier- und Zeigeobjekten (86, 88), Tangententafeln (100), elektronischen und computerunterstützten Meß- und Auswertemitteln (66, 68, 76, 78, 80) sowie mit Einrichtungen (40, 60) zur Überwachung der Kopf- und/oder Augapfel-Position, **dadurch gekennzeichnet, daß** in einem Hüllraum (30) für einen Probanden eine Darstellungsfläche (20) vorhanden ist, auf der rechnergestützt eine virtuelle Tangententafel (100) und koordinatenbezogene Seheindrücke (E) freisichtig darbietbar sind.

22. Anlage nach Anspruch 21, **dadurch gekennzeichnet, daß** Einrichtungen wie fernbedienbare elektronische Hilfsmittel (32), Touchscreen (56), Tastenfeld, Steuerknüppel (Joystick), Mikrofon (52) u.dgl. zur interaktiven Beeinflussung von Seheindrücken (E) und Verfahrensschritten durch den Untersucher und/oder den Probanden vorhanden sind.

23. Anlage nach Anspruch 21 oder 22, **dadurch gekennzeichnet, daß** die virtuelle Tangententafel (100) und/oder die Seheindrücke (E) auf einem Computermonitor, Großbildschirm o.dgl. erzeugbar sind, vorzugsweise durch Bilddaten eines Computers und/oder Videorecorders.

24. Anlage nach einem der Ansprüche 21 bis 23, **dadurch gekennzeichnet, daß** sie zum Erzeugen der virtuellen Tangententafel (100) und/oder der Seheindrücke (E) ein Projektionssystem (12) mit Einrichtungen für Aufprojektion und/oder Rückprojektion aufweist.

25. Anlage nach einem der Ansprüche 21 bis 24, **dadurch gekennzeichnet, daß** innerhalb des Hüllraumes (30) zumindest ein Laser-Projektor (90, 92) vorhanden ist, jedoch nicht an einer Kopfstütze oder als deren Bestandteil.

26. Anlage nach einem der Ansprüche 21 bis 25, **gekennzeichnet** t durch Mittel zur Darbietung des Koordinatensystems der virtuellen Tangententafel (100) in Verbindung mit einem Hintergrundbild und/oder mit einer gegenständlichen Tangententafel.

27. Anlage nach einem der Ansprüche 21 bis 26, **gekennzeichnet durch** Mittel zur Darbietung beliebiger Seheindrücke (E) in Verbindung mit der virtuellen Tangententafel (100).

28. Anlage nach einem der Ansprüche 21 bis 27, **dadurch gekennzeichnet, daß** Mittel (99) zur automatischen Protokollierung einschließlich grafischer und/oder numerischer Aufzeichnung (102) sowie Wiedergabe vorhanden sind.

29. Anlage nach einem der Ansprüche 21 bis 28, **gekennzeichnet durch** Mittel (76, 78, 80) zur Überwachung der objektiven Stellung eines Augapfels oder beider Augäpfel des Probanden und zur Zuordnung seiner subjektiven Seheindrücke wie Schielwinkel in ein und derselben Messung, beispielsweise **durch** Infrarotkameras (80) mit Aufnahme von Hornhaut-Reflexbildern (R).

30. Anlage nach einem der Ansprüche 21 bis 29, **gekennzeichnet durch** Mittel (74) zur blickgetrennten Darbietung von Fixierobjekten (86, 88), namentlich in Form eines frequenzsteuerbaren Shutters, mit dem alternierend bzw. wahlweise ein linker und ein rechter Sichtkanal bzw. ein linkes und ein rechts Teilbild okkludierbar ist.

31. Anlage nach einem der Ansprüche 21 bis 30, **dadurch gekennzeichnet, daß** zur automatischen Feststellung der Kopfhaltung und ihrer Änderungen am Probandenkopf (K) Trägerstücke oder -flächen für Meßwert-Geber und/oder -Sensoren (66) eines Kontrollsystems (60) vorhanden bzw. anbringbar sind, mit dem die Untersuchung bei Erreichen eines vorgegebenen Unterschieds zwischen Soll- und Ist-Parameterwert unterbrechbar ist.

32. Anlage nach Anspruch 31, **dadurch gekennzeichnet, daß** das Kontrollsystem (40) ein stirnseitig ansetzendes Band, einen Klemmstreifen (42) o.dgl. mit einem an oder nahe dessen hinterem Ende zum Ohrenbereich führenden Querstreifen (44) aufweist.

33. Anlage nach einem der Ansprüche 21 bis 32, **dadurch gekennzeichnet, daß** eine integrierte akustischen Begleitsteuerung vorhanden ist, d.h. eine Vorgabe an die Anlage (10) seitens einer Person z.B. mit Mikrofonen, Lauthöreinrichtungen usw., und daß Mittel für eine in Abhängigkeit von der laufenden Untersuchung synthetisch erzeugten und/oder digitalisierten Sprachführung vorhanden sind, d.h. eine Anweisung der Anlage (10) an Personen, nämlich für den Probanden und/oder den Bediener.

34. Anlage nach einem der Ansprüche 21 bis 33, **dadurch gekennzeichnet, daß** zur Beobachtung sowie grafischen und/oder numerischen Registrierung der Kopfhaltung Videografie-Einrichtungen vorhanden sind, insbesondere Infrarot-Kameras (80) und -Strahler (78).

35. Anlage nach einem der Ansprüche 21 bis 34, **dadurch gekennzeichnet, daß** ein separater Bedienerbildschirm (54) zur Darstellung eines zusätzlichen Untersucherbildes vorhanden ist.

36. Anlage nach einem der Ansprüche 21 bis 35, **dadurch gekennzeichnet, daß** eine optoelektronische Einrichtung (90...99) zum wahlweisen oder zusätzlichen Abtasten von an einer Tangententafel (100) dargebotenen Seheindrücken (E) und zu deren abrufbarer Speicherung vorhanden ist.

## Claims

1. . Method for carrying out vision tests especially on proband visual disorders such as strabismus by using projection installations, visual impression transmitters, fixing-and indicator light, tangent boards as well as electronic and computer-aided measuring and analyzing devices, with head and/or eyeball positions control, **characterized by** a virtual tangent board being generated under computer control, to which coordinate-related visual impressions are presented at free sight.

2. . Method according to claim **characterised by** the presentation surface being arranged with the virtual tangent board in an enveloping cubicle surrounding the proband.

3. . Method according to claim 1 or 2, **characterized**

4. . Method according to one of the claims 1 to 3, **characterized by** the virtual tangent board and/or the visual impressions being generated on a computer monitor, a large screen or the like.

5. . Method according to one or the claims 1 to 4, **characterized by** the image data of a computer and/or video recorder being projected.

6. . Method according to one of the claims 1 to 5, **characterized by** the virtual tangent board and/or the visual impressions being generated with help of a projection system by front projection and/or rear projection,

7. . Method according to one of the claims 2 to 6, **characterised by** at least one laser projector being used for the presentation and/or positioning or visual impressions on a tangent board within the enveloping cubicle, but not on a head-rest or as integral part of it.

8. . Method according to one of the claims 1 to 7, **characterized by** the tangent board coordinate system being calculated according to variable parameters such as origin displacement,viewer distance, proband head position, etc., especially in a computer unit by using algorithms, functional equations, and the like.

9. . Method according to one of the claims 1 to 8, **characterised by** the coordinate system of the virtual tangent board being combined with a background image and/or a solid tangent board.

10. . Method according to one of the claims 1 to 9, **characterized by** optional visual impressions being used in combination wit the virtual tangent board.

11. . Method according to one of the claims 1 to 10, **characterised by** the measuring results being recorded automatically and ready for print by graphic and/or numeric recording.

12. . Method according to one of the claims 1 to 11, **characterized by** the test at a virtual tangent board being combinable with other visual direction-dependent visual tests e.g. of monocular or binocular viewing, acuity test, etc. with the optotypes being movable over the complete field of view and display.

13. . Method according to one of the claims 1 to 12, **characterized by** the objective position of one eyeball or both eyeballs of the proband in a test at a tangent board being controlled and within one and the same measuring process assigned to his subjective viewing impressions, e.g. the subjective strabismus angles.

14. . Method according to claim 12 or 13, **characterized by** the control of the focussing movements of the eyes of the proband being done by the separate and alternating presentation of objects of fixation, with the vieweing impressions on the virtual tangent board being so long moved or newly positioned until no more eye focussing movement occurs.

15. . Method according to one of the claims 1 to 14, **characterized by** the focussing movements of one eye or both eyes being automatically captured and the simultaneous or alternating objective strabismus angles measured at the tangent board being assigned to the subjective deviations, optionally spot-related to one of the eyes or both eyes or to the middle between them (root of the nose).

16. . Method to carry out visual tests, especially at proband visual deviations such as strabismus by using projection installations, visual impression transmitters, fixing and indicator light, tangent boards, as well as electronic and computer-controlled measuring and analysing means, with head-and/or eyeball position control according to one of the claims to 15, **characterized by** a coordinate system being analytically related to the papillary centre of each eye with tests at free-sight view and by the origins of each coordinate system being optionally displaced according to the eyebase of the proband or united coincidentally in the middle between the eyes (root of the nose).

17. . Method according to one of the claims 1 to 15, **characterized by** a control system automatically indicating the test performer deviations between the set and actual head position of a proband during test at a tangent board, with the control system being applied to the free movable or fixed head of the proband especially interrupting the test when a preset parameter difference between the set and actual head position is reached or exceeded.

18. . Method according to one of the claims 1 to 16, **characterized by** the test being accompanied by an integrated acoustic control, that is, an instruction to the apparatus by a person by using for example microphones, speakerphone devices, etc. and by providing a voice-guidance synthetically produced and/or digitalized according to the test under way, that is, an instruction by the apparatus for persons, namely the proband and/or the operator.

19. . Method according to one of the claims to 17, **characteerized** by a separate operator screen to display a test performer picture being used in addition to the tangent board picture the proband sees.

20. . Method according to one of the claims 1 to 10, **characterized by** the visual impressions presented on a tangent board being scanned by a photo-electronic device and stored ready for recall.

21. . Apparatus (10) for carrying out visual tests especially on proband visual disorders such as strabismus, with projection installations (12), visual impression transmitters (96, 32), fixing and indicator objects (86, 88), tangent hoards (100), electronic and computer-aided measuring and analyzing, devices (66, 68, 76, 78, 80), as well as installations (40, 60) to control head and/or eyeball positions, **characterized by** having in an enveloping cubicle (30) a presentation surface (20) for a proband on which by computer control a virtual tangent board (100) and coordinate-related visual impressions (E) can be presented at free sight.

22. . Apparatus according to claim 21, **characterized by** installations such as remote-controllable electronic means (32), touch-screen (56), keyboard, control stick (joystick), microphone (52), and the like being at hand for the interactive influence of visual impressions (E) and test steps by the test performer and/or the proband.

23. . Apparatus according to claim 21 or 22, **characterized by** the virtual tangent board (100) and/or the visual impressions (E) being generatable on a computer monitor, large screen or the like, preferably by the image data of computer and/or video recorder.

24. . Apparatus according to one of the claims 21 to 23, **characterized by** possessing for the generation of the virtual tangent board (100) and/or the visual impressions (E) a projection system (12) with installations for front projection and/or rear projection.

25. . Apparatus according to one of the claims 21 to 24, **characterized by** at least one laser projector (90, 92) existing within the enveloping cubicle (30), but not on the head-rest or as an integral part of it.

26. . Apparatus according to one of the claims 21 to 25, **characterized by** means for the presentation of the coordinate system of the virtual tangent board (100) in connection with a background image and/or a solid tangent board.

27. . Apparatus according to one of the claims 21 to 26, **characterized by** means for the presentation or optional visual impressions (E) in connection with the virtual tangent board (100).

28. . Apparatus according to one of the claims 21 to 27, **characterized by** having means (99) for the automatic recording including the graphic and/or numeric recording (102) and reproduction.

29. . Apparatus according to one of the claims 21 to 28, **characterized by** means (76, 78, 80) for the control the objective position of an eyeball or of both eyeballs of the proband and the assignment of his subjective visual impressions such as strabismus angles within one and the same measuring process, c. g. by infrared cameras (80) by taking cornea ghost images (R),

30. . Apparatus according to one of the claims 21 to 29, **characterized by** means (74) for the view-split presentation of fixing objects (86), 88), namely in the form of a frequency-controlled shutter by which alternatingly or optionally a right or left visual channel respectively a left or a right partial image is occludable.

31. . Apparatus according to one of the claims 21 to 30, **characterized by** receptacle pieces or-surfaces for data transmitters and/or -sensors (66) of a control system (60) for the automatic determination of the automatic determination of the head position or its changes are present, respectively attachable at the proband's head (K) by which the test is interruptable when a preset difference between the set and actual parameter value is reached.

32. . Apparatus according to claim 31, **characterized by** the control system (40) consisting of a forehead headband, a clamp strap (42) or the like, with a cross strap (44) at or near its rear end leading to the ear area.

33. . Apparatus according to one of the claims 21 to 32, **characterized by** having an integrated associate acoustic control, that is, an instruction to the apparatus (10) by a person for example by microphones,speakerphone devices, etc, and by providing means for a synthetically produced and/or digitalized voice-guidance according to the test under way, that is, an instruction by the apparatus (10) for persons, namely the proband and/or the operator.

34. . Apparatus according to one of the claims 21 to 33,**characterized by** having for the observation and graphic and/or numeric registration of the head position videography installations, especially infrared cameras (80) and beamers (78).

35. . Apparatus according to one of the claims 21 to 34, **characterized by** having a separate operator's screen (54) to display an additional test performer picture

36. . Apparatus according to one of the claims 21 to 35, **characterized by** a photo-electronic device (90...99) for the optional or additional scanning of visual impressions (E) displayed on a tangent board (100) and for their ready-for-call storage.

## Revendications

1. Méthode destinée à la réalisation d'examens de la vision, notamment en cas de troubles visuels de sujets, tels que le strabisme, en utilisant des dispositifs de projection, des transmetteurs d'impressions visuelles, de la lumière de fixation et de la lumière par pointeur, des tableaux de tangentes ainsi que des moyens de mesure et d'évaluation électroniques et assistés par ordinateurs, avec contrôle de la position de la tête et/ou du globe oculaire, **caractérisée en ce qu'**un tableau de tangentes virtuel est créé, de manière commandée par ordinateur, sur une surface de représentation, vers lequel tableau des impressions visuelles relatives à des coordonnées sont représentées en vue libre,

2. Méthode selon la revendication 1, **caractérisée en ce que** la surface de représentation comprenant le tableau de tangentes virtuel est disposée dans un espace enveloppant, entourant le sujet.

3. Méthode selon la revendication 1 ou 2, **caractérisée en ce que**, pendant l'examen, des impressions visuelles et des étapes de la méthode sont influençables interactivement par l'examinateur et/ou par le sujet, à savoir à l'aide de moyens électroniques à télécommande, d'écran tactile, panneau de touches, manette de commande (joystick), microphone et similaires.

4. Méthode selon l'une quelconque des revendications 1 à 3, **caractérisée en ce que** l'on crée le tableau de tangentes virtuel et/ou les impressions visuelles sur un écran d'ordinateur, un grand écran ou similaires.

5. Méthode selon l'une quelconque des revendications 1 à 4, **caractérisée en ce que** des données d'image d'un ordinateur et/ou d'un magnétoscope sont projetées.

6. Méthode selon l'une quelconque des revendications 1 à 5, **caractérisée en ce que** l'on crée le tableau de tangentes virtuel et/ou les impressions visuelles à l'aide d'un système de projection, par projection frontale et/ou par rétroprojection.

7. Méthode selon l'une quelconque des revendications 2 à 6, **caractérisée en ce que**, pour représenter et/ou positionner les impressions visuelle sur un tableau de tangentes à l'intérieur de l'espace enveloppant, on utilise au moins un projecteur à laser, cependant pas sur un appui-tête ni en tant que partie constitutive de cet appui-tête.

8. Méthode selon l'une quelconque des revendications 1 à 7, **caractérisée en ce que** l'on calcule le système de coordonnées du tableau à tangentes selon des paramètres variables, tels que déplacement de l'origine, distance par rapport à l'observateur, position de la tête du sujet, etc., notamment dans une unité d'ordinateur, en utilisant des algorithmes, des équations fonctionnelles et similaires.

9. Méthode selon l'une quelconque des revendications 1 à 8, **caractérisée en ce que** l'on combine le système de coordonnées du tableau de tangentes virtuel à une image d'arrière-plan et/ou à un tableau de tangentes concret.

10. Méthode selon l'une quelconque des revendications 1 à 9, **caractérisée en ce que**, en relation avec le tableau de tangentes virtuel, on utilise des impressions visuelles quelconques.

11. Méthode selon l'une quelconque des revendications 1 à 10, **caractérisée en ce que** l'on archive les résultats de mesure automatiquement et de manière imprimable par enregistrement graphique et/ou numérique.

12. Méthode selon l'une quelconque des revendications 1 à 11, **caractérisée en ce que** d'autres examens de la vision dépendant de la direction du regard, par ex. de la vision monoculaire ou binoculaire, test d'acuité visuelle, etc., sont combinés à l'examen réalisé sur un tableau de tangentes virtuel, les symboles visuels étant déplaçables sur tout le champ visuel et le champ de vision.

13. Méthode selon l'une quelconque des revendications 1 à 12, **caractérisée en ce que**, lors d'un examen réalisé sur un tableau de tangentes, la position objective d'un globe oculaire ou des deux globes oculaires du sujet est contrôlée et **en ce que**, dans une seule et même mesure, elle est associée à ses impressions visuelles subjectives, par ex. aux angles de déviation subjectifs.

14. Méthode selon la revendication 12 ou 13, **caractérisée en ce que** le contrôle de mouvements d'ajustement des yeux du sujet est effectué par représentation séparée et alternante d'objets de fixation, les impressions visuelles étant déplacées ou repositionnées sur le tableau de tangentes virtuel jusqu'à ce qu'il n'y ait plus de mouvement d'ajustement des yeux.

15. Méthode selon l'une quelconque des revendications 1 à 14, **caractérisée en ce que** des mouvements d'ajustement d'un oeil ou des deux yeux sont enregistrés automatiquement et **en ce que** les angles de déviation objectifs simultanés ou alternants, mesurés sur le tableau de tangentes, sont associés aux déviations subjectives, au choix par rapport à un point sur l'un des yeux ou sur les deux yeux ou au milieu entre les yeux (racine du nez).

16. Méthode destinée à la réalisation d'examens de la vision, notamment en cas de troubles visuels de sujets, tels que le strabisme, en utilisant des dispositifs de projection, des transmetteurs d'impressions visuelles, de la lumière de fixation et de la lumière par pointeur, des tableaux de tangentes ainsi que des moyens de mesure et d'évaluation électroniques et assistés par ordinateurs, avec contrôle de la position de la tête et/ou du globe oculaire, selon l'une quelconque des revendications 1 à 15, **caractérisée en ce que**, dans les examens en vue libre, un système de coordonnées fait référence au milieu de la pupille, de manière séparée pour chaque oeil par voie de calcul, et **en ce que** les origines de chaque système de coordonnées sont, au choix, déplacées les unes par rapport aux autres de l'écart entre les yeux du sujet ou réunies de manière à coïncider au milieu entre les yeux (racine du nez).

17. Méthode selon l'une quelconque des revendications 1 à 15, **caractérisée en ce que** des écarts entre la position souhaitée et la position réelle de la tête d'un sujet sont automatiquement affichés à l'examinateur par un système de contrôle pendant un examen réalisé sur un tableau de tangentes, le système de contrôle placé sur la tête du sujet, librement mobile ou fixée, interrompant l'examen notamment lorsqu'une différence paramétrique prédéfinie entre la position souhaitée et la position réelle de la tête est atteinte ou dépassée,

18. Méthode selon l'une quelconque des revendications 1 à 16, **caractérisée en ce que** l'examen est accompagné d'une commande acoustique intégrée, c'est-à-dire d'une indication donnée au dispositif de la part d'une personne, par ex. en utilisant des microphones, des dispositifs à haut-parleurs, etc., et **en ce que**, en fonction de l'examen en cours, on prévoit un guide vocal créé synthétiquement et/ou un guide vocal numérique, c'est-à-dire une instruction du dispositif à des personnes, à savoir pour le sujet et/ou l'opérateur.

19. Méthode selon l'une quelconque des revendications 1 à 17, **caractérisée en ce qu'**un écran d'opérateur à part, destiné à la représentation d'une image pour l'examinateur, est utilisé en plus de l'image du tableau de tangentes vue par sujet.

20. Méthode selon l'une quelconque des revendications 1 à 10, **caractérisée en ce que** les impressions visuelles représentées sur un tableau de tangentes sont balayées par un dispositif optoélectronique et **en ce qu'**elles sont mémorisées de manière à pouvoir être appelées.

21. Dispositif (10) destiné à la réalisation d'examens de la vision, notamment en cas de troubles visuels de sujets, tels que le strabisme, comprenant des dispositifs de projection (12), des transmetteurs d'impressions visuelles (96, 32), des objets de fixation et des objets à montrer (86, 88), des tableaux de tangentes (100) ainsi que des moyens de mesure et d'évaluation (66, 68, 76, 78, 80) électroniques et assistés par ordinateurs, et comprenant des dispositifs (40, 60) destinés au contrôle de la position de la tête et/ou du globe oculaire, **caractérisé en ce que** dans un espace enveloppant (30), une surface de représentation (20) est présente pour un sujet, sur laquelle un tableau de tangentes virtuel (100) et des impressions visuelles (E) relatives à des coordonnées sont représentables en vue libre de manière assistée par ordinateur.

22. Dispositif selon la revendication 21, **caractérisé en ce que** des équipements, tels que des moyens électroniques télécommandables (32), écran tactile (56), panneau de touches, manette de commande (joystick), microphone (52) et similaires, sont présents pour l'influence interactive d'impressions visuelles (E) et d'étapes de la méthode par l'examinateur et/ou le sujet.

23. Dispositif selon la revendication 21 ou 22, **caractérisé en ce que** le tableau de tangentes virtuel (100) et/ou les impressions visuelles (E) peuvent être générés sur un écran d'ordinateur, sur un grand écran ou similaires, de préférence au moyen de données d'image d'un ordinateur et/ou d'un magnétoscope.

24. Dispositif selon l'une quelconque des revendications 21 à 23, **caractérisé en ce que**, pour générer le tableau de tangentes virtuel (100) et/ou les impressions visuelles (E), il présente un système de projection (12) comprenant des dispositifs pour projection frontale et/ou pour rétroprojection.

25. Dispositif selon l'une quelconque des revendications 21 à 24, **caractérisé en ce qu'**au moins un projecteur à laser (90, 92) est présent à l'intérieur de l'espace enveloppant (30), cependant pas sur un appui-tête ni en tant que partie constitutive de cet appui-tête.

26. Dispositif selon l'une quelconque des revendications 21 à 25, **caractérisé par** des moyens destinés à la représentation du système de coordonnées du tableau de tangentes virtuel (100), en relation avec une image d'arrière-plan et/ou un tableau de tangentes concret.

27. Dispositif selon l'une quelconque des revendications 21 à 26, **caractérisé par** des moyens destinés à représenter des impressions visuelles quelconques (E) en relation avec le tableau de tangentes virtuel (100).

28. Dispositif selon l'une quelconque des revendications 21 à 27, **caractérisé en ce que** des moyens (99) destinés à l'archivage automatique, y compris l'enregistrement graphique et/au numérique (102), ainsi qu'à la restitution sont présents.

29. Dispositif selon l'une quelconque des revendications 21 à 28, **caractérisé par** des moyens (76, 78, 80) destinés au contrôle de la position objective d'un globe oculaire ou des deux globes oculaires du sujet et destinés à associer ses impression visuelles subjectives, telles que l'angle de déviation, dans une seule et même mesure, par exemple par des caméras à infrarouge (80) avec prise d'images réflexes de la cornée (R).

30. Dispositif selon l'une quelconque des revendications 21 à 29, **caractérisé par** des moyens (74) destinés à la représentation, par division du regard, d'objets de fixation (86, 88), à savoir sous forme d'un obturateur réglable en fréquence, au moyen duquel, en alternance ou au choix, un canal visuel gauche et un canal visuel droite ou une image partielle gauche et image partielle droite peuvent être occlus.

31. Dispositif selon l'une quelconque des revendications 21 à 30, **caractérisé en ce que** pour constater automatiquement la position de la tête et ses modifications, des pièces de support ou des surfaces de support sont présentes ou peuvent être placées sur la tête (K) du sujet pour des transmetteurs de valeurs de mesure et/ou pour des capteurs de valeurs de mesure (66) d'un système de contrôle (60), au moyen duquel l'examen peut être interrompu lorsqu'une différence prédéfinie entre une valeur paramétrique souhaitée et une valeur paramétrique réelle est atteinte.

32. Dispositif selon la revendication 31, **caractérisé en ce que** le système de contrôle (40) présente une bande à appliquer du côté du front, une bande à serrer (42), ou similaires, munie d'une bande transversale (44) sur son extrémité arrière ou proche de son extrémité arrière menant vers la partie de l'oreille.

33. Dispositif selon l'une quelconque des revendications 21 à 32, **caractérisé en ce qu'**une commande d'accompagnement acoustique intégrée est présente, c'est-à-dire une indication au dispositif (10) de la part d'une personne, par ex. par microphones, dispositifs à haut-parleurs, etc., et **en ce que**, en fonction de l'examen en cours, des moyens pour un guide vocal créé synthétiquement et/ou un guide vocal numérique sont présents, c'est-à-dire une instruction du dispositif à des personnes, à savoir pour le sujet et/ou l'opérateur.

34. Dispositif selon l'une quelconque des revendications 21 à 33, **caractérisé en ce que** pour l'observation ainsi que pour l'enregistrement graphique et/ou numérique de la position de la tête, des dispositifs de vidéographie sont présents, notamment des caméras à infrarouge (80) et des émetteurs de rayonnement infrarouge (78).

35. Dispositif selon l'une quelconque des revendications 21 à 34, **caractérisé en ce qu'**un écran d'opérateur à part (54) est présent pour la représentation d'une image supplémentaire pour l'examinateur.

36. Dispositif selon l'une quelconque des revendications 21 à 35, **caractérisé en ce qu'**un dispositif optoélectronique (90...99) est présent pour le balayage facultatif ou supplémentaire d'impressions visuelles (E) représentées sur un tableau de tangentes (100) et pour leur mémorisation par appel.
